# FASCICULE DE BREVET EUROPEEN

(11) **EP 1 526 875 B1**
(45) Date de publication et mention de la délivrance du brevet: **27.05.2009**
(21) Numéro de dépôt: 03771011.8
(22) Date de dépôt: 24.07.2003
(51) Int. Cl.: A61L 2/14

(54) **PROCEDE DE HAUTE PERFORMANCE POUR LA STERILISATION PAR PLASMA GAZEUX**
LEISTUNGSSTARKES VERFAHREN ZUR PLASMASTERILISIERUNG
HIGH PERFORMANCE METHOD FOR STERILISING BY GASEOUS PLASMA

(30) Priorité: 26.07.2002 CA 2395659
(43) Date de publication de la demande: 04.05.2005
(73) Titulaire: Valorisation-Recherche, Limited Partnership, Montreal, QC H3X 2H9 (CA)
(72) Inventeur: MOISAN, Michel, Outremont, Québec H2V 2Z1 (CA); PHILIP, Nicolas, France (FR); SAOUDI, Bachir, Montréal, Québec H4A 2K3 (CA)
(74) Mandataire: Dronne, Guy
(86) Numéro de dépôt international: PCT/CA2003/001116
(87) Numéro de publication internationale: WO 2004/011039

(56) Documents cités:
- WO-A-00/72889
- FR-A- 2 654 000
- US-A- 3 948 601
- US-A- 5 325 020

## Description

### DOMAINE DE L'INVENTION

La présente demande est relative à un dispositif et à un procédé de haute performance permettant la stérilisation d'objets, notamment d'instruments et accessoires médicaux, par mise en oeuvre d'un plasma gazeux, aussi appelé gaz ionisé. Ce procédé et ce dispositif permettent de réaliser, en un seul traitement, la stérilisation d'une surface importante d'objets contaminés.

### ART ANTÉRIEUR

En raison de normes de plus en plus strictes imposées pour la stérilisation des objets notamment dans le domaine médical et dans le domaine alimentaire, il existe un besoin croissant pour de nouveaux dispositifs hautement performants et dépourvus des limitations et/ou des inconvénients des dispositifs connus.

Les dispositifs mettant en oeuvre des procédés de stérilisation par plasma, malgré leurs excellentes performance, n'ont pas encore réussi à faire une percée significative dans le marché de la stérilisation traditionnellement occupé par des dispositifs mettant en oeuvre des traitements à la vapeur et/ou des traitements chimiques.

Le document WO-A-00/72889 décrit la stérilisation par une post-décharge de plasma, cette décharge s'effectue dans un mélange gazeux, avec comme gaz principal soit de l'azote, soit de l'argon. Un tel mélange est décrit comme devant avantageusement contenir entre 0,5 % et 20 % de O₂, afin d'obtenir le temps de stérilisation le plus court possible.

L'effet des UV sur l'ADN est étudié dans l'article de Moisan et al. dans International Journal of Pharmaceutics, vol. 226 pp. 1-21 (2001).

Ces procédés de l'art antérieur présentent des limitations notamment au niveau de l'homogénéité de la distribution des espèces stérilisantes et, en conséquence à l'endroit des objets contaminés et au niveau de la surface totale des objets contaminés qu'il est possible de traiter dans un même enceinte.

Il existait donc un besoin pour un nouveau procédé dépourvu d'au moins une limitation des dispositifs de l'art antérieur.

### DESCRIPTION SOMMAIRE DES DESSINS

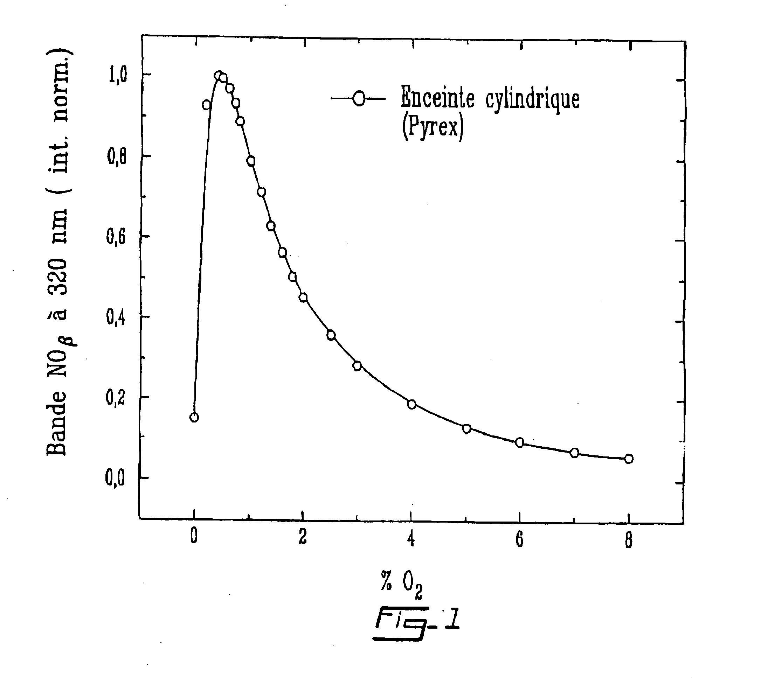

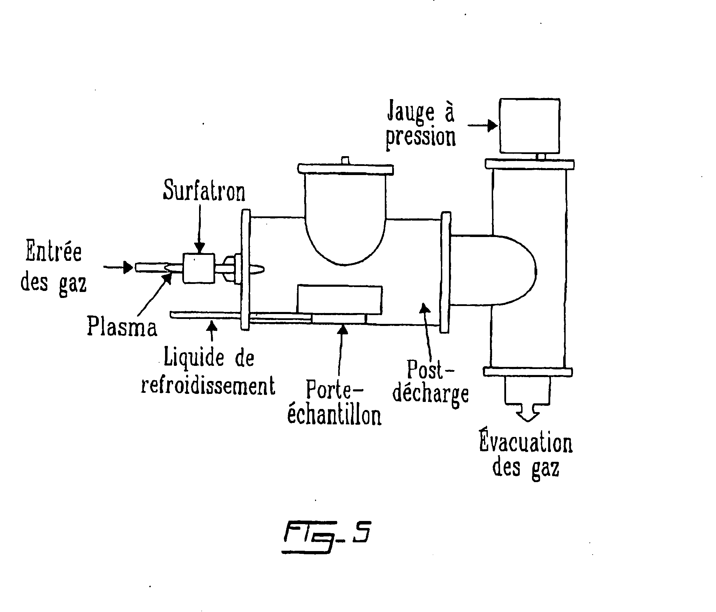

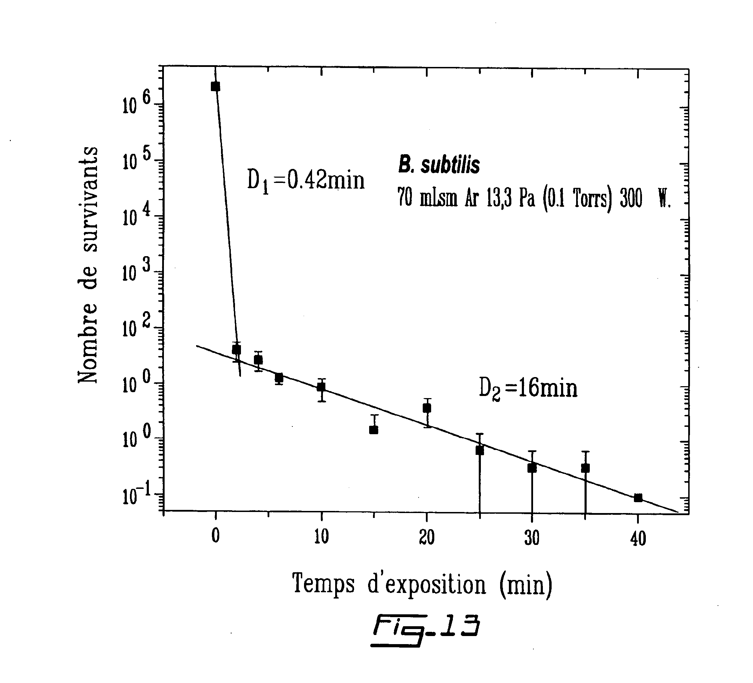

- **La Figure 1/13**: représente la variation de l'intensité d'émission des photons UV, mesurée à 320 nm dans la bande NO_{β} (250-380 mm), en fonction du pourcentage de O₂ ajouté à N₂ dans le mélange N₂-O₂ alimentant le conduit de décharge d'une enceinte de stérilisation cylindrique, selon le dispositif représenté sur la Figure 5/13 de 20 litres, muni d'un tube à décharge dont le diamètre intérieur est de 6 mm, et d'un générateur haute fréquence émettant une onde dont la fréquence est 2.450 MHz à une puissance de 100 W, à une pression de 665 Pa (5 Torrs), et à un débit de 1 litre standard/minute (1sm).

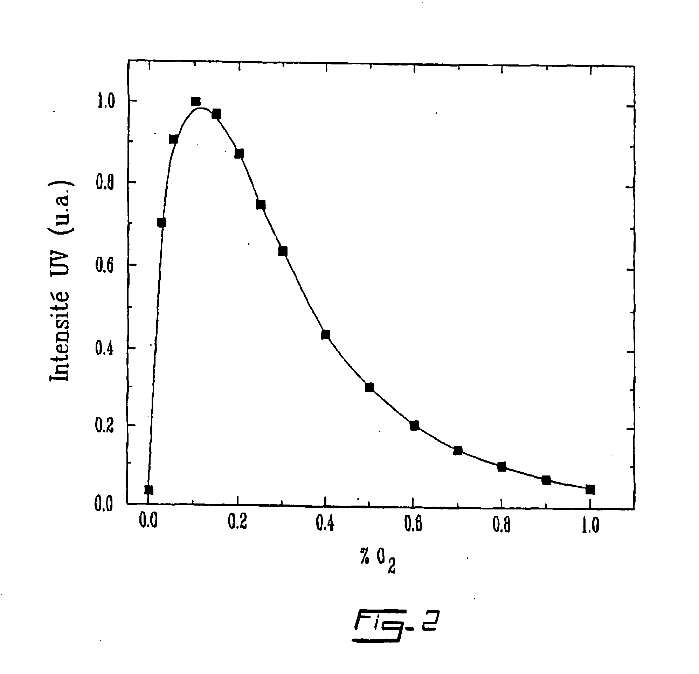

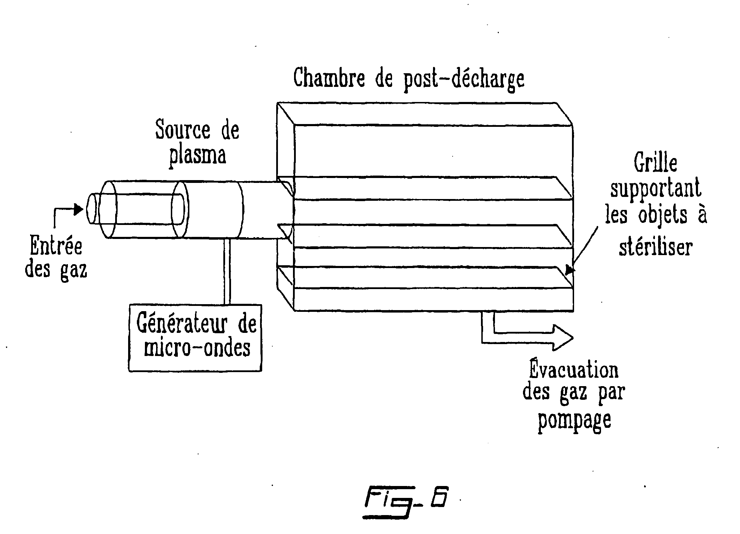

- **La Figure 2/13**: représente la variation de l'intensité d'émission des photons UV, mesurée à 320 nm dans la bande NO_{β} (250-380 nm), en fonction du pourcentage de O₂ ajouté à N₂ dans le mélange N₂-O₂ alimentant le conduit de décharge d'une enceinte de stérilisation parallélépipédique de 50 litres, selon le dispositif représenté sur la Figure 6/13, muni d'un tube à décharge d'un diamètre intérieur de 26 mm, et d'un générateur haute fréquence émettant une onde dont la fréquence est de 915 MHz par une puissance de 300 W, à une pression de 798 Pa (6 Torrs), 2 lsm.

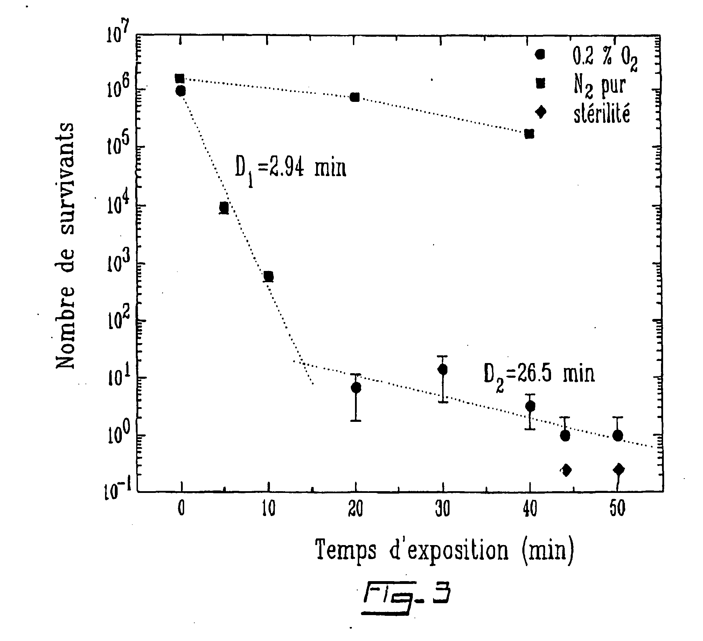

- **La Figure 3/13**: représente un diagramme de survivants de spores *B. subtilis* soumises à une post-décharge de N₂-O₂ à 0 et 0,2 % de O₂ ajouté à N₂. La stérilisation est obtenue dans l'enceinte de la Figure 6/13, en environ 40 minutes, à 0,2% de O₂ selon la méthode des tests positifs ou négatifs (symbole losange). Le temps D indiqué pour chaque phase représente le temps nécessaire pour réduire la population de spores de 90%. Avec une pression dans l'enceinte de 1064 Pa (8 Torrs), avec un débit de 2 lsm, et avec une puissance de 300 W.

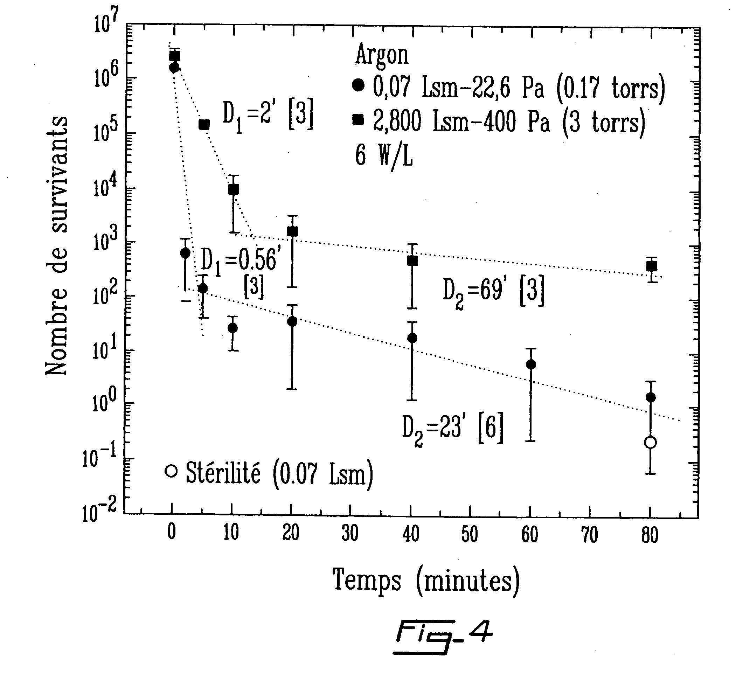

- **La Figure 4/13**: représente un diagramme de survivants réalisé dans une post-décharge d'argon pur, aux pressions de 23 et 400 Pa (0,17 et 3 Torrs). La stérilité est obtenue dans l'enceinte représentée dans la Figure 6/13 en environ 40 minutes, selon la méthode du test positif ou négatif (losange), à la pression de 23 Pa (0,17 Torrs), alors qu'il reste encore plus de 500 spores à inactiver à 400 Pa (3 Torrs).
- **La Figure 5/13**: montre le dispositif utilisé dans le cadre du procédé objet de la demande PCT/CA00/00623: la chambre de stérilisation, de géométrie cylindrique, est en Pyrex et d'une contenance de 20 litres environ. Le plasma est produit par une décharge micro-ondes à 2.450 MHz dans un tube de diamètre interne de 6 mm avec R=0,04.
- **La Figure 6/13**: présente un dispositif de stérilisation conforme à la présente invention: la chambre de stérilisation, de forme parallélépipédique, est en aluminium avec des fenêtres d'observation (pour effectuer les mesures de spectroscopie optique) en silice fondue et d'une contenance de 50 litres (les stérilisateurs classiques font 50, 100 ou 150 litres). L'uniformité des espèces actives dans la chambre de stérilisation, qui assure que les objets soient stérilisés, quelle que soit leur position dans la chambre, et qui minimise les effets de turbulence à haut débit de gaz (on peut fonctionner alors à 2-3 lsm sans turbulence), a pu être obtenue grâce à un tube de diamètre de décharge élargi à 26 mm (pour ce faire, la fréquence micro-ondes a également été abaissée de 2.450 MHz à 915 MHz (R=0,09)). En effet, le premier dispositif de la Figure 5/13 (R= 0,04) n'était vraiment efficace qu'au voisinage de l'axe du tube à décharge.

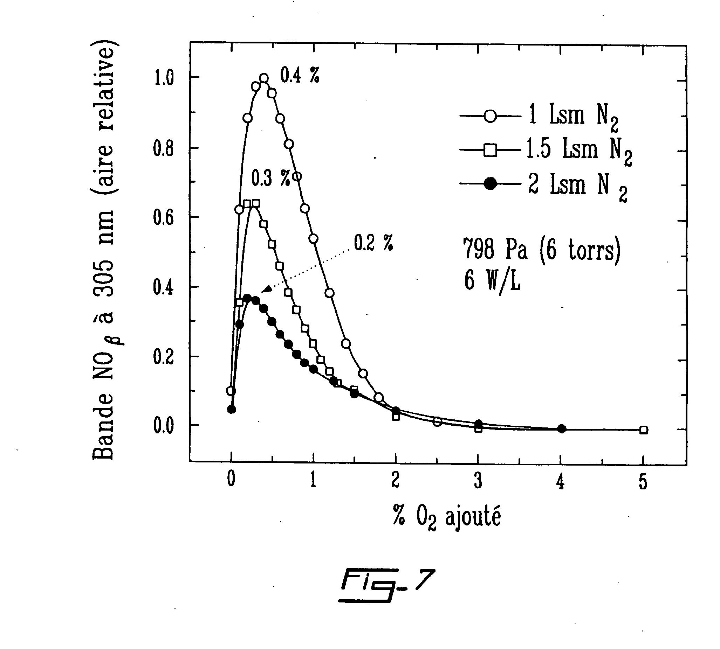

- **La Figure 7/13**: présente le profil de rémission UV en fonction du pourcentage d'oxygène moléculaire ajouté à N₂, à différents débits. Sur chaque courbe est précisé le pourcentage optimum d'oxygène. L'intensité du signal UV est collectée à l'entrée de l'enceinte de la Figure 6/13.

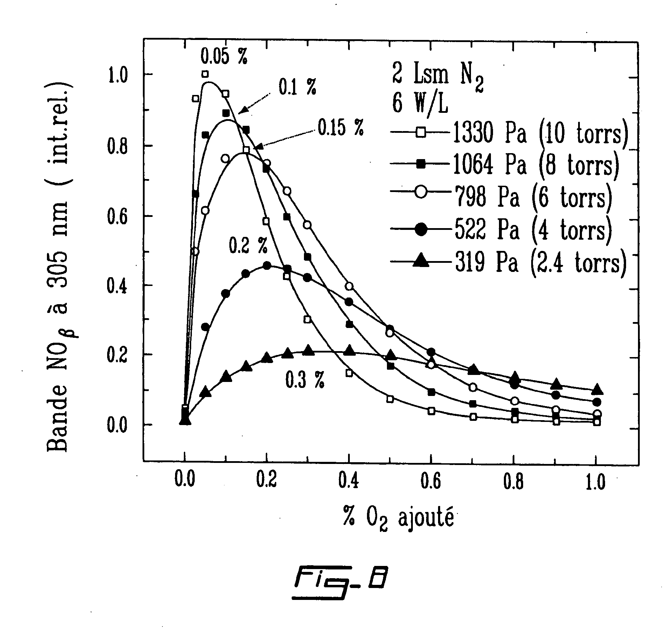

- **La Figure 8/13**: représente le profil de l'émission UV en fonction du pourcentage d'oxygène moléculaire ajouté à N₂, à différentes pressions, dans une enceinte de stérilisation selon la Figure 6/13.

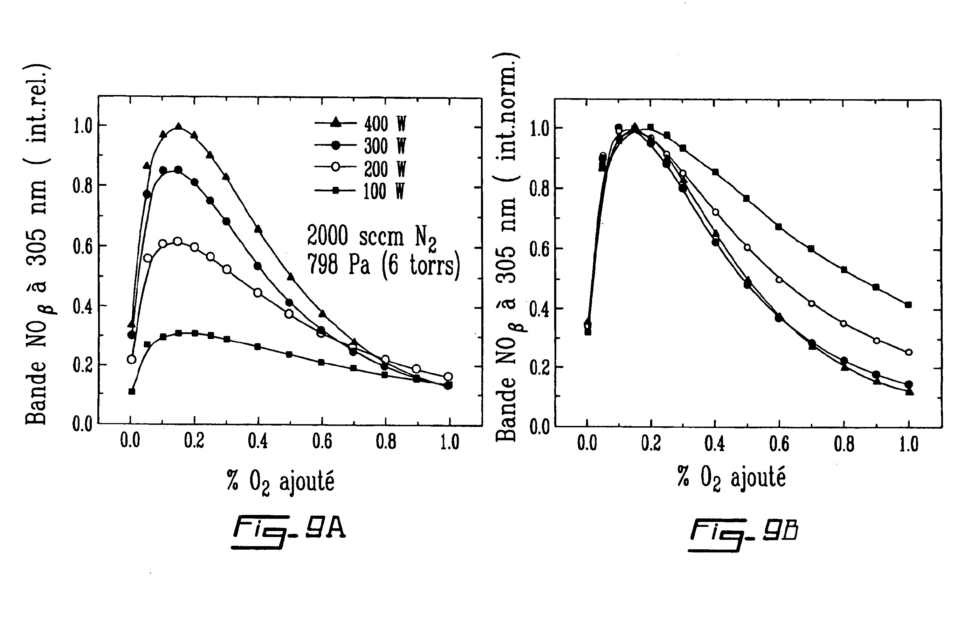

- **La Figure 9/13**: montre l'influence, dans une enceinte de stérilisation selon la Figure 6/13, de la puissance micro-ondes sur les profils d'émission UV, en fonction du pourcentage d'oxygène ajouté à N₂: (a) intensité relative et (b) les mêmes intensités normalisées.

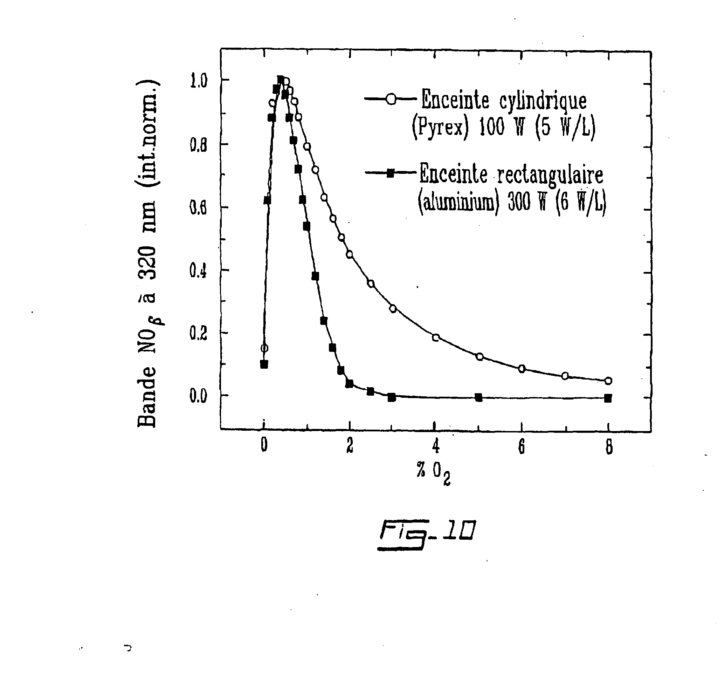

- **La Figure 10/13**: présente les profils comparés de l'émission UV selon le pourcentage d'oxygène dans les deux enceintes; 1 Lsm N₂ - 665 Pa (5 torrs); enceinte cylindrique (Figure 5/13, 100 W, 5 W/L) *vs* enceinte parallélépipédique (Figure 6/13 300 W, 6 W/L). Les intensités sont normalisés à l'unité.

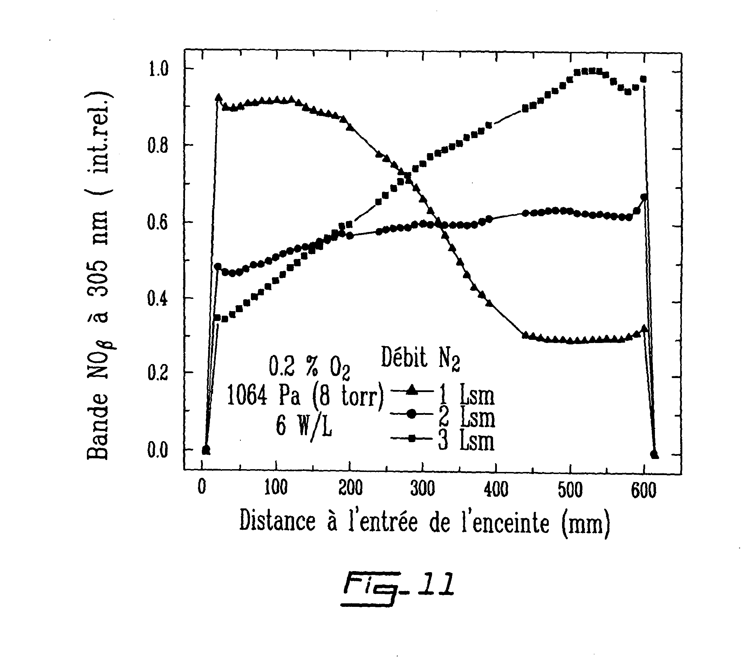

- **La Figure 11/13**: décrit la variation de l'intensité de l'émission UV le long de l'axe de la décharge dans l'enceinte de la Figure 6/13, pour différents débits d'azote, à pourcentage d'oxygène ajouté fixe (0,2%) et à pression constante. Autour de 2 Lsm, l'intensité est relativement uniforme le long de l'enceinte.

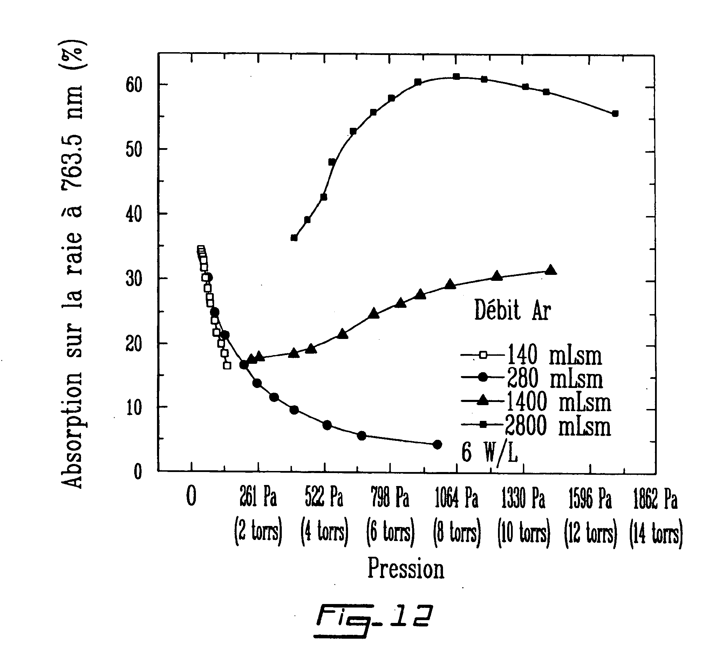

- **La Figure 12/13**: montre que la pression à laquelle on observe, dans une enceinte de stérilisation selon la Figure 6/13, le maximum en intensité des UV dépend beaucoup du débit d'argon. Si celui-ci est faible (e.g. 70 mlsm), la pression de fonctionnement doit être faible (ici autour de 13,3 Pa (0.1 Torr)). Au contraire, s'il est grand, la pression optimale sera plus élevée.
- **La Figure 13/13**: montre une courbe de survie de spores *B. subtilis* dans une post-décharge d'argon pur, dans une enceinte de stérilisation selon la Figure 6/13, obtenue dans les conditions indiquées, et en présence d'une intensité d'émission UV proche de la valeur maximale.

### RÉSUMÉ DE L'INVENTION

L'invention concerne un procédé de stérilisation d'objets par exposition, dans un dispositif ayant des caractéristiques géométriques particulières, à l'aide d'un plasma généré dans un gaz d'une composition spécifique.

### DESCRIPTION GÉNÉRALE DE L'INVENTION

Il a été trouvé de façon surprenante que pour une même enceinte de stérilisation, et pour une même fréquence, la surface d'objets à stériliser peut considérablement être augmentée par une sélection de certains paramètres structuraux et énergétiques du système.

Il a été également établi que l'homogénéité axiale du plasma est influencée par le débit et par la pression, ainsi qu'éventuellement par la concentration en oxygène du gaz d'alimentation utilisé pour la production du plasma.

Il a par ailleurs été trouvé que l'homogénéité transversale et axiale du flux de plasma dépend d'un rapport structural R, significatif de la différence de taille du tube de décharge par rapport à celle de l'enceinte de stérilisation, R est ci-après défini. Pour augmenter l'homogénéité transversale notamment, il est nécessaire d'accroître le rapport R.

Le rapport R est limité supérieurement par le diamètre du tube à décharges utilisé. Le diamètre maximal du tube à décharge augmente lorsqu'on abaisse la fréquence du champ électrique. D'autre part, il existe une limite inférieure en fréquence du champ électrique quant à l'efficacité du transfert de puissance HF vers la décharge. Par conséquent, il est avantageux d'harmoniser le diamètre du tube et la fréquence de la source de plasma de manière à obtenir la plus grande homogénéité possible des espèces stérilisantes.

Un objet de la présente invention est constitué par un procédé de stérilisation d'objets contaminés comprenant l'exposition, dans une enceinte de stérilisation, des dits objets à un plasma généré dans au moins un conduit de décharge arrivant dans ladite enceinte. À partir d'un flux gazeux de N₂, le plasma obtenu comprend des espèces stérilisantes générées lors de la soumission du flux à un champ électrique suffisamment intense pour générer le plasma.

Le procédé comprend l'exposition des objets contaminés aux espèces stérilisantes, cette exposition a lieu dans une zone de post-décharge ou dans une zone d'excitation du plasma.

Ce procédé est **caractérisé en ce que:**
- le pourcentage d'oxygène moléculaire ajouté au flux de N₂ gazeux est ajusté à une teneur x, en oxygène moléculaire, telle que 0 < x < 0,5% (x variant préférentiellement de 0,1 à 0,4 %), de préférence par réglage du débit et/ou par réglage de la pression du gaz dans l'enceinte, de façon à obtenir un rayonnement UV d'intensité maximale;
- l'oxygène moléculaire est transformé, au moins partiellement en oxygène atomique; et
- la section du conduit de décharge, à son entrée dans la chambre de stérilisation (SCD) et la section de la chambre de stérilisation (SCS), vérifient la relation 0,05 < (SCD)/(SCS) < 0,7, de préférence 0,20 < (SCD)/(SCS) < 0,5, plus préférentiellement encore (SCD)/(SCS) est environ 0,25,
la section (SCD) représente la section du conduit de décharge au contact de la chambre de stérilisation et qui est perpendiculaire à la direction du flux gazeux alimentant le conduit de décharge et la section (SCS) représentant la section de la chambre au contact du conduit de décharge et qui est perpendiculaire au courant de plasma.

L'enceinte de stérilisation présente de préférence une section perpendiculaire mais aussi parallèle à la direction du flux de plasma entrant qui est quasi constante.

Le champ électrique qui génère le plasma est de préférence un champ à haute fréquence dont la fréquence est habituellement comprise entre 10 Mégahertz et 3 Gigahertz, de préférence cette fréquence est comprise entre 100 et 2.450 MHz, plus préférentiellement encore cette fréquence varie de 200 à 915 MHz.

Afin d'obtenir un rayonnement Ultra Violet d'intensité maximale et homogène, le flux gazeux d'alimentation est ajusté par réglage du débit et/ou de la pression du gaz dans l'enceinte.

Ainsi, le débit du flux gazeux d'alimentation est ajusté à une valeur comprise entre 10 et 5.000 cm³ par minute, plus préférentiellement encore à une valeur comprise entre 10 et 3.000 cm³ par minute.

De façon avantageuse, la pression générée à l'intérieur de l'enceinte de stérilisation est comprise entre 13,3 Pa et 1330 Pa (0,1 Torrs et 10 Torrs).

Selon une variante particulièrement intéressante, le flux gazeux d'alimentation comprend de l'argon et la pression générée à l'intérieur de l'enceinte de stérilisation est comprise entre 13,3 Pa et 532 Pa (0,1 et 4 Torrs).

Selon une autre variante préférentielle, le flux gazeux comprend de l'azote et de l'oxygène moléculaire et la pression générée à l'intérieur de l'enceinte de stérilisation est comprise entre 133 Pa et 1064 Pa (1 et 8 Torrs).

Des résultats intéressants sont également obtenus lorsque le flux gazeux d'alimentation comporte au moins un composant choisi dans le groupe constitué par l'oxygène moléculaire, l'azote, le néon, l'argon, le krypton, le xénon, l'hélium, l'oxygène, le monoxyde de carbone, le dioxyde de carbone, les gaz de formule NOₓ, avec x représentant un nombre entier sélectionné dans le groupe constitué par 1, 2 ou 3, l'air, et les mélanges d'au moins deux ou de plusieurs de ces derniers.

De préférence, le flux gazeux d'alimentation comporte de l'oxygène moléculaire. Dans ce cas, le flux gazeux comporte avantageusement au moins 0,04 % d'oxygène moléculaire, plus préférentiellement encore au moins 0,10 % d'oxygène moléculaire.

Selon une variante intéressante, le flux gazeux d'alimentation comporte, en plus de l'oxygène moléculaire, au moins deux autres gaz.

Selon une autre variante intéressante, le flux gazeux comporte, en plus de l'oxygène moléculaire, au moins trois autres gaz. Ainsi, le flux gazeux peut avantageusement comporter, en plus de l'oxygène moléculaire et de l'azote, de l'argon et de l'hélium, de l'argon et du dioxyde d'azote ou bien du xénon ou du krypton.

De façon préférentielle, le débit du gaz d'alimentation est compris entre 10 et 5.000 cm³ standard par minute, plus préférentiellement encore ce débit est compris entre 50 et 3.000 cm³ par minute.

A titre illustratif, le flux gazeux peut être constitué de NO₂, d'azote, ou d'un mélange oxygène - azote et la pression générée à l'intérieur de l'enceinte de stérilisation est alors avantageusement comprise entre 266 Pa et 1064 Pa (2 et 8 Torrs).

Des compositions très variables sont possibles pour les mélanges ternaires d'alimentation, sous forme liquide et/ou gazeuse. Ainsi les compositions suivantes sont utilisables à savoir de 0,04 à 30 %d'O₂ de 0,05 à 99, 85 % d'azote et de 0,05 à 99,85 % d'argon ou de krypton.

Des compositions très variables sont également possibles pour les mélanges quaternaires sous forme gazeuse et/ou liquide. Ainsi, les compositions suivantes sont utilisables à savoir de 0,04 à 99,85 % d'O₂, de 0,05 à 99,85 % d'azote, de 0,05 à 99,85 % de xénon et/ou de 0,05 à 99,85 % de néon.

Selon un mode préférentiel, les mélanges d'alimentation peuvent comporter de 0,1 à 10 % d'O₂, plus préférentiellement encore de 0,2 à 5 % d'O₂.

Plus préférentiellement, le champ électrique utilisé pour la décharge est généré par une décharge micro-ondes dont la fréquence d'excitation (est de préférence située dans la bande autorisée pour fins industrielles, médicales et scientifiques (ISM) varie habituellement de 915 à 2.450 MHz.

Ce procédé permet de produire des espèces stérilisantes comprenant des photons, des radicaux, des atomes et/ou des molécules. De préférence, dans ces mélanges d'espèces stérilisantes, la population de photons et/ou de radicaux est majoritaire.

Avantageusement, le flux gazeux comprend, en plus de l'oxygène moléculaire, de l'azote, de l'argon et leurs mélanges.

Ce procédé est particulièrement performant pour le traitement des objets contaminés par des micro-organismes tels que les virus, spores, bactéries, champignons et moisissures et par des prions.

De préférence, lors de la mise en oeuvre de ce procédé, la température dans l'enceinte de stérilisation ne dépasse pas 60° Celsius, et de préférence cette température est d'environ 30° Celsius et la durée du traitement des objets contaminés est comprise entre 10 minutes et 4 heures.

Ce procédé peut être mis en oeuvre de façon isolée ou répétitive selon un processus séquentiel à plusieurs étapes. Ainsi les étapes peuvent inclure l'utilisation d'un gaz pulsé dans un champ électrique appliqué de façon continue, dans un champ électrique pulsé dans un gaz en flux continu, dans un gaz pulsé, dans un champ électrique pulsé de façon synchrone. Un changement de gaz, en cours de processus, est également envisageable de même qu'une combinaison des étapes particulières précédemment énumérées.

Le dispositif permettant la mise en oeuvre du procédé selon l'invention comporte une source de plasma couplée, au niveau d'une des parois, à une chambre de stérilisation, par au moins un tube à décharge, dans lequel est injecté un gaz ou un mélange de gaz générant éventuellement le plasma, la chambre comprend des objets à stériliser, et une pompe à vide pour entraîner les gaz dans la chambre et y maintenir une pression réduite. La source de plasma comprend un applicateur de champ électrique, de préférence à haute fréquence et le rapport R = (SCD)/(SCS), dans lequel (SCD) représente la section du conduit de décharge ou la somme des sections des conduit(s) de décharge au contact de la chambre de stérilisation et (SCS) la section de la chambre de stérilisation, vérifie la relation 0,05 < R < 0,7.

La chambre de stérilisation est de préférence de forme cylindrique ou parallélépipedique, et avantageusement cette chambre de stérilisation est de sections quasi-constantes.

### DESCRIPTION DE MODES PRÉFÉRENTIELS DE L'INVENTION

Il a été découvert de façon surprenante que des performances très intéressantes pouvaient être obtenues avec des plasmas formés à partir d'un mélange de gaz ayant une teneur en O₂ pouvant être aussi faible que 0,4 %, notamment de gaz à forte teneur en N_{2,} dans le cas d'un tube à décharge de 6 mm de diamètre intérieur (Figure 5/13). Dans le cas où un tube de plus grand diamètre, soit 26 mm de diamètre intérieur, est utilisé, le pourcentage optimal en O₂ peut être aussi faible que 0,1% (Figure 2/13). Cependant, si la teneur est presque égale à 0%, comme le montre la Figure 3/13, la stérilisation ne s'obtient alors qu'après un temps très long (non déterminé).

Des résultats particulièrement intéressants ont été également obtenus avec un tube à décharge de plus grand diamètre (26 mm au lieu de 6 mm), et en travaillant à une fréquence micro-ondes de 915 MHz, au lieu de 2.450 MHz.

Alors que dans la WO-A-00/72889, l'utilisation d'une décharge d'argon seul ne conduit pas à la stérilisation dans un temps raisonnable, comme le mettent en évidence les figures de ce document de l'art antérieur. Ces résultats avaient été obtenus à une pression de travail comprise entre 42 le et 931 Pa (3,2 et 7 Torrs).

Il est à noter que la pression de travail représente un paramètre important pour l'optimisation de l'intensité d'émission des photons UV dans une décharge d'argon. Comme le montrent notamment les résultats répertoriés dans la Figure 4/13, une pression voisine de 26.6 Pa (0,2 Torr) maximise l'intensité d'émission des photons UV provenant des états résonnants de l'argon pur excité.

Le diamètre du tube et le volume de l'enceinte (chambre) de stérilisation représentés dans les Figures 1/13 et 5/13 sont respectivement de 6 mm et 20 litres et pour les Figures 2/13, 3/13, 4/13 , 6/13 et suivantes (sauf 10/13) sont de 26 mm et de 50 litres. La Figure 10/13 est une comparaison des mesures de l'intensité UV dans les deux enceintes (Figures 5/13 et 6/13).

À partir d'une certaine longueur du tube de décharge, l'uniformité des espèces stérilisantes augmente.

La longueur d'onde des UV influe sur le taux d'inactivation de l'ADN et aussi sur les dommages causés aux objets à stériliser. La longueur d'onde des UV croît dans l'ordre suivant (pour les gaz rares): hélium, néon, argon, krypton, xénon.

Lorsque mentionné, le débit indiqué est ramené aux conditions standard de pression c'est-à-dire à une atmosphère et à une température de 298 K.

À titre d'illustration de l'effet sur la stérilisation, la Figure 4/13 montre le diagramme de survivants de spores *B. subtilis* soumises à une post-décharge d'argon, aux pressions de 22,6 et 400 Pa (0,17 et 3 Torrs). L'optimisation du rayonnement UV, obtenue en ajustant la pression de travail autour de 13,3-22.6 Pa (0,1 - 0,2 Torr), permet une stérilisation en environ 40 minutes.

L'uniformité de la distribution spatiale des photons UV émis dans l'enceinte de stérilisation a été par ailleurs améliorée en agissant sur le débit du gaz considéré. Il s'est avéré que le débit de gaz, ainsi fixé, peut affecter sensiblement la pression optimale de fonctionnement en ce qui a trait au maximum d'émission des photons UV.

L'optimisation de l'intensité du rayonnement UV s'obtient dans le cas d'un gaz monoatomique (argon, y compris tous les gaz rares comme hélium, néon, krypton, xénon) en faisant varier la pression du gaz dans la chambre de stérilisation. À titre d'exemple, le domaine de pression sur lequel s'exerce l'optimisation s'étend de 13,3 à 1330 Pa (0,1 à 10 Torrs) dans le cas de l'argon.

Le recours à des gaz rares purs, évitant l'oxygène, devrait permettre de stériliser en causant moins d'érosion aux objets à traiter.

### Description d'un dispositif de stérilisation par plasma dans une forme commerciale

Le dispositif pour la mise en oeuvre du procédé selon l'invention comprend trois éléments essentiels: la source de plasma, la chambre de stérilisation où l'on dispose (par exemple, sur des grilles) les objets à stériliser, et une pompe à vide pour : 1) évacuer initialement la chambre jusqu'à une pression résiduelle d'environ 2,7-6,7 Pa (20-50 mTorr), et 2) maintenir ensuite la pression désirée en présence du débit de gaz de la décharge en assurant en même temps le renouvellement des espèces actives par évacuation continuelle des gaz.

Les chambres de stérilisation testées on été construites en aluminium, matériau léger et peu coûteux. Leur forme peut être cylindrique ou parallélépipédique.

Des moyens de support des objets à stériliser sont intégrés à la chambre, et celle-ci est avantageusement munie d'une porte d'accès permettant d'effectuer le chargement et le déchargement du stérilisateur.

Les sources de plasma : 1) assurent un rayonnement UV suffisant pour pouvoir stériliser en au plus 40 minutes; 2) garantissent l'uniformité des espèces actives dans la chambre. À cette fin, on maintiendra un diamètre maximal du tube à décharge par rapport à la section de la chambre (diamètre qui fixe la fréquence supérieure de travail). On peut recourir à plus d'une source de plasma, celles-ci étant disposées adéquatement, et on ajuste le débit de gaz pour optimiser cette uniformité; 3) évitent de dépasser 50 à 60 °C dans la chambre; et 4) utilisent des gaz et des conditions opératoires qui minimisent les dommages causés aux objets par ce procédé de stérilisation (en particulier, utiliser le moins de O₂ possible dans les mélanges de gaz).

Le système de stérilisation comprend un dispositif assurant le cycle de stérilisation : l'évacuation de l'enceinte, l'arrivée des gaz, le réglage de la pression et du débit, l'alimentation en puissance micro-ondes et l'allumage de la décharge; en fin de cycle, le retour à la pression atmosphérique. Pour contrôler le bon fonctionnement du stérilisateur, on y effectue une vérification des paramètres physiques (vide primaire, pression et débit de gaz, puissances micro-ondes incidente et réfléchie, intensité du rayonnement UV dans l'enceinte en au moins deux points et enregistrement de cette intensité tout au long du cycle plasma). Enfin, on imprime, en fin de cycle, les paramètres du traitement avec traçabilité des objets stérilisés.

### EXEMPLES

Les exemples suivants de mise en oeuvre du procédé selon l'invention sont donnés à titre illustratif et ne sauraient en aucun cas être interprétés comme constituant une quelconque limitation de l'objet de l'invention.

Les résultats qui suivent ont été obtenus dans la chambre de stérilisation de forme parallélépipédique (50 litres), à l'exception des Figures 1/13, 5/13 et 10/13 (en partie).

La mesure de l'uniformité de distribution du rayonnement UV dans l'enceinte de stérilisation est réalisée dans les exemples de la façon suivante.

L'enceinte de stérilisation (Figure 6/13) est dans le cas présent munie d'une fenêtre en silice fondue, transparente aux UV au-delà de 180 nm. La lumière émise par un élément de volume de la post-décharge est collectée, via un collimateur, par une fibre optique reliée à la fente d'entrée du spectromètre optique. Le spectromètre optique (à réseau) permet d'enregistrer l'intensité de l'émission UV de la post-décharge à une longueur d'onde donnée. L'ensemble collimateur-fibre optique est conçu de façon à pouvoir se déplacer dans un plan suivant les axes X et Y, permettant ainsi d'établir la cartographie, longitudinalement et transversalement, de l'intensité de l'émission UV (enregistrée habituellement à 320 nm).

### 1) Maximum d'émission UV sur la bande NO_{β} en fonction du pourcentage de O₂ dans le mélange N₂-O₂.

Les résultats de cette section 1) sont obtenus, sauf mention, dans la chambre de la Figure 6/13 comportant un seul conduit de décharge et alimentée par un mélange gazeux constitué de N₂ et de O₂. Le rapport R=0,09.

### Variation du pourcentage optimal de O₂ avec le débit

Les résultats rapportés sur la Figure 7/13 montrent le profil de l'émission UV en fonction du pourcentage d'oxygène ajouté, à différents débits. Sur chaque courbe, il est précisé le pourcentage optimum oxygène. L'intensité du signal est collectée à l'entrée de l'enceinte.

On constate que le pourcentage optimal d'oxygène moléculaire, pour des débits de gaz réalistes, se situe entre 0,1 est 0,4% O₂ ajouté à N₂ pour former le mélange N₂-O₂.

### Variation du pourcentage optimal de O₂ avec la pression

Les résultats rapportés dans la Figure 8/13: montrent le profil de l'émission UV en fonction du pourcentage d'oxygène ajouté à différentes pressions.

Pour un débit de 2 lsm, le pourcentage optimal se situe entre 0.05% O₂ (1330Pa) (10 Torrs) et 0,3% 319 Pa (2,4 Torrs) où les pressions considérées sont réalistes.

### Variation du pourcentage optimal de O₂ avec la puissance micro-ondes absorbée dans la décharge et rapportée à la contenance de l'enceinte de stérilisation (2 à 8 W/litre)

Les résultats rapportés dans la Figure 9/13 mettent en évidence l'influence de la puissance micro-ondes utilisée sur les profils d'émission UV en fonction du pourcentage d'oxygène (a) intensités relatives; (b) la mêmes intensités, normalisées.

On constate que, pour une pression et un débit de gaz donnés, la puissance dissipée dans la décharge influe peu sur le pourcentage optimal en O₂.

### Variation du pourcentage optimal de O₂ avec la configuration et le volume de l'enceinte

Les deux enceintes, étudiées de façon comparative dans le présent exemple, sont celles représentés dans les Figures 5/13 et 6/13.

Les résultats rapportés dans la Figure 10/13 montrent les profils comparés de l'émission UV selon le pourcentage d'oxygène dans les deux enceintes: 1 Lsm N₂ - 665 Pa - (5 torrs); enceinte en Pyrex (5 W/L) *vs* enceinte parallélépipédique (6 W/L). Les intensités sont normalisées.

L'étude du profil de variation de l'intensité d'émission UV en fonction du pourcentage de O₂ ajouté à N₂ montre un maximum pour le même pourcentage dans les deux enceintes (indépendance des paramètres opératoires, à savoir fréquence micro-ondes, diamètre du tube à décharge, géométrie et nature de l'enceinte, rapport R, position de l'orifice de pompage, ...); par contre, la décroissance de l'intensité UV s'effectue moins rapidement dans l'enceinte cylindrique.

À noter que l'enceinte cylindrique (Figure 5/13) est alimentée par une décharge à 2450 MHz alors que l'enceinte parallélépipédique (Figure 6/13) l'est par une décharge à 915 MHz.

Conclusion : dans les exemples montrés, le pourcentage optimal se situe entre 0,05 et 0,4% de O₂ ajouté à N₂ couvrant ainsi une grande plage de variation des paramètres opératoires (fréquence micro-ondes, débit, diamètre du tube à décharge, ...).

### 2) Amélioration de l'uniformité de la distribution spatiale des espèces émettrices de photons UV dans l'enceinte de stérilisation

Les résultats qui suivent ont été obtenus dans l'enceinte parallélépipédique de 50 litres représentée de façon schématique dans la Figure 6/13. Les mesures ont été effectuées par spectroscopie optique d'émission à la longueur d'onde habituelle (320 nm) représentative de la bande NO_{β}.

Les résultats rapportés dans la Figure 11/13 représentent la variation de l'intensité de l'émission UV le long de l'axe de la décharge dans l'enceinte rectangulaire, pour différents débits d'azote, à pourcentage d'oxygène ajouté fixe (0.2%) et à pression constante. Autour de 2 Lsm, l'intensité des UV est relativement uniforme le long de l'enceinte.

Pour une pression donnée à (ou au voisinage immédiat de) l'optimum de O₂ pour l'émission UV, le réglage du débit de N₂ (ici à 2 lsm) permet d'obtenir une émission UV uniforme le long de l'enceinte (l'arrivée du plasma est située sur la gauche de la Figure 11/13).

Si l'on veut obtenir l'uniformité pour un autre débit, il faut modifier en conséquence la pression dans l'enceinte.

### 3) Inactivation des spores par une post-décharge d'argon pur

Les résultats rapportés dans la Figure 12/13 montrent l'absorption de la raie d'argon à 763.5 nm en fonction de la pression pour différents débits.

Les mesures sont effectuées à 5 cm de l'entrée de l'enceinte à l'aide d'un spectromètre optique.

L'intensité des raies VUV de l'argon dépend de la densité des états résonnants qui émettent ces photons. On obtient la densité de ces états par des mesures d'absorption optique: plus l'absorption est grande, plus l'intensité VUV émise sera grande.

La façon la plus directe d'optimiser l'émission UV dans le cas d'un gaz unique comme l'argon pur est de faire varier la pression. La Figure 12/13 montre effectivement que la pression à laquelle on observe le maximum en intensité des UV dépend beaucoup du débit d'argon. Si celui-ci est faible (e.g. 70 mlsm), la pression de fonctionnement doit être faible (ici autour de 13,3 Pa (0.1 Torr). Au contraire, s'il est grand, la pression optimale sera plus élevée.

La courbe de survie de spores *B.* subtilis est représentée sur la Figure 13/13 et confirme les résultats de la Figure 4/13.

L'ajustement de la pression et du débit d'argon permettent d'obtenir le meilleur rendement au niveau de l'émission UV, ce qui permet de réaliser la stérilisation en un temps raisonnable.

Par ailleurs, les résultats rapportés dans la Figure 13/13 montrent la courbe de survie des spores *B. subtilis* dans une post-décharge d'argon pur, obtenue dans les conditions indiquées, et en présence d'une intensité d'émission UV proche de la valeur maximale.

Un troisième stérilisateur (non représenté), conforme à la présente invention, de charpente parallélépipédique en aluminium, et de 60 litres de contenance, est alimenté par un plasma produit à 200 MHz dans un tube de 48 mm de diamètre intérieur. Les résultats obtenus sont comparables à ceux recueillis avec le stérilisateur parallélépipédique de 50 litres fonctionnant à 915 MHz. Pour accroître l'uniformité de distribution des espèces actives dans la chambre de stérilisation, celle-ci est alimentée à partir de deux sources de plasma, séparées de façon optimale l'une de l'autre. Par ailleurs, l'orifice d'évacuation des gaz est placé, dans le cas présent, dans l'axe de la chambre, alors que dans la Figure 6/13 le pompage s'effectue par le bas de la chambre.

## Revendications

1. Procédé de stérilisation d'objets contaminés comprenant l'exposition, dans une chambre de stérilisation, desdits objets à un plasma généré dans au moins un conduit de décharge arrivant dans ladite chambre, a partir d'un flux gazeux d'azote comprenant de l'oxygène moléculaire, ledit plasma comprenant des espèces stérilisantes générées lors de la soumission dudit flux gazeux à un champ électrique suffisamment intense pour générer le plasma, ledit procédé comprenant l'exposition des objets contaminés aux espèces stérilisantes, ladite exposition ayant lieu dans une zone de post-décharge ou dans une zone d'excitation du plasma, dans lequel :
- le pourcentage d'oxygène moléculaire dans le flux d'azote gazeux est ajusté à une teneur x, en oxygène moléculaire, telle que 0 < x < 0,5%, x variant préférentiellement de 0,1 à 0,4%, et un rayonnement UV d'intensité maximale est obtenu en réglant le débit dudit flux gazeux pour une pression donnée ou en réglant la pression dudit flux gazeux pour un débit donné;
- l'oxygène moléculaire est transformé au moins partiellement en oxygène atomique; et
- la section du conduit de décharge à son entrée dans la chambre de stérilisation (SCD) et celle de la chambre de stérilisation (SCS) vérifient la relation 0,05 < (SCD)/(SCS) < 0,7,
la section (SCD) représentant la section du conduit de décharge au contact de la chambre de stérilisation et qui est perpendiculaire à la direction du flux gazeux alimentant le conduit de décharge et la section (SCS) représentant la section de la chambre au contact du conduit de décharge et qui est perpendiculaire au courant de plasma.

2. Procédé selon la revendication 1, dans lequel 0,09 ≤ R ≤ 0,60, de préférence 0,15 ≤ R ≤ 0,5.

3. Procédé selon la revendication 2, dans lequel 0,2 ≤ R ≤ 0,40, de préférence R est voisin de 0,25.

4. Procédé selon l'une quelconque des revendications 1 à 3, dans lequel le champ électrique que génère le plasma est un champ à haute fréquence.

5. Procédé salon la revendication 4, dans lequel la fréquence de champ électrique est comprise entre 10 Mégahertz et 3 Gigahertz, de préférence la fréquence varie de 100 à 2450 MHz.

6. Procédé selon la revendication 5, dans lequel ladite fréquence est comprise entre 200 et 915 MHz.

7. Procédé selon l'une quelconque des revendications 1 à 6, dans lequel le débit du flux gazeux d'alimentation a une valeur qui est comprise entre 10 et 5000 cm³ standard par minute.

8. Procédé selon la revendication 7, dans lequel le débit du flux gazeux d'alimentation est compris entre 50 et 3000 cm³ standard par minute.

9. Procédé selon l'une quelconque des revendications 1 à 8, dans lequel la pression générée à l'intérieur de la chambre de stérilisation est comprise entre 13,3 et 1330 Pa (0,1 et 10 Torrs).

10. Procédé selon la revendication 9, dans lequel la pression générée à l'intérieur de la chambre de stérilisation est comprise entre 133 et 1064 Pa (1 et 8 Torrs).

11. Procédé de stérilisation selon l'une quelconque des revendications 1 à 10, dans lequel le flux gazeux d'alimentation comporte en plus de l'azote et de l'oxygène moléculaire au moins un autre gaz.

12. Procédé de stérilisation selon l'une quelconque des revendications 1 à 10, dans lequel le flux gazeux comporte, en plus de l'azote et de l'oxygène moléculaire, au moins deux autres gaz.

13. Procédé de stérilisation selon la revendication 11 ou 12, dans lequel les autres gaz sont choisis parmi l'argon, l'hélium, le dioxyde d'azote, le xénon, le néon et le krypton.

14. Procédé selon l'une quelconque des revendications 1 à 13, dans lequel les espèces stérilisantes comprennent des photons, des radicaux, des atomes et/ou des molécules.

15. Procédé selon la revendication 14, dans lequel la population de photons et/ou de radicaux est majoritaire.

16. Procédé selon l'une quelconque des revendications 1 à 15, dans lequel les objets sont contaminés par des micro-organismes tels que les virus, spores, bactéries, champignons, moisissures et par les prions.

17. Procédé selon l'une quelconque des revendications 1 à 16, dans lequel la température dans la chambre de stérilisation est de 60° Celsius ou moins, et de préférence cette température est d'environ 30° Celsius.

18. Procédé selon l'une quelconque des revendications 1 à 17, d'une durée comprise entre 10 minutes et 4 heures.

19. Procédé selon l'une quelconque des revendications 1 à 18, réalisé de façon isolée ou répétitive dans un processus séquentiel à plusieurs étapes.

20. Procédé selon la revendication 19, dans lequel les étapes mettent en oeuvre un gaz pulsé dans un champ électrique appliqué de façon continu, un champ électrique pulsé dans un gaz en flux continu, un gaz pulsé dans un champ électrique pulsé de façon synchrone, un changement de gaz; ou une combinaison de ces étapes.

## Claims

1. Process for the sterilisation of contaminated objects whereby said objects are exposed, in a sterilisation chamber, to plasma generated in at least one discharge duct opening into said chamber using a nitrogen gas stream containing molecular oxygen, wherein said plasma contains the sterilising species generated by subjecting said gas stream to a sufficiently intense electrical field to generate the plasma, said process comprising exposure of contaminated objects to sterilising species, said exposure taking place in a post-discharge zone or plasma excitation zone wherein:
- the percentage of molecular oxygen in the gas stream is adjusted to a content x, molecular oxygen, such as 0 < 0.5%, x preferably ranging from 0.1 to 0.4%, and maximum intensity UV radiation is obtained by regulating the flow rate of said gas stream for a given pressure or by regulating pressure of said gas stream for a given flow rate;
- molecular oxygen is transformed at least partially into atomic oxygen; and
- the section of the discharge duct at the inlet to the sterilisation chamber (DDS) and that of the sterilisation chamber (SCS) correspond to the proportion 0.05 < (DDS)/(SCS) < 0.7,
the section (DDS) representing the discharge duct section in contact with the sterilisation chamber which is perpendicular to the direction of the gas stream supplies the discharge duct and the section (SCS) representing the chamber section in contact with the discharge duct which is perpendicular to the plasma current.

2. Process according to claim 1 wherein 0.09 ≤ R ≤ 0.60, preferably 0.15 ≤ R ≤ 0.5.

3. Process according to claim 2 wherein 0.2 ≤ R ≤ 0.40, preferably R is around 0.25.

4. Process according to any one of claims 1 to 3 wherein the electrical field which generates the plasma is a high frequency field.

5. Process according to claim 4 wherein the frequency of the electrical field is between 10 Megahertz and 3 Gigahertz, preferably the frequency is between 100 and 2450 MHz.

6. Process according to claim 5 wherein said frequency is between 200 and 915 MHz.

7. Process according to any one of claims 1 to 6 wherein the supply gas stream has a flow rate between 10 and 5000 cm³ standard per minute.

8. Process according to claim 7 wherein the supply gas stream is between 50 and 3000 cm³ standard per minute.

9. Process according to any one of claims 1 to 8 wherein the pressure generated inside the sterilisation chamber is between 13.3 and 1330 pa (0.1 and 10 Torrs).

10. Process according to claim 9 wherein the pressure generated inside the sterilisation chamber is between 133 and 1064 Pa (1 and 8 Torrs).

11. Sterilisation process according to any one of claims 1 to 10 wherein the supply gas stream consists of at least one other gas in addition to nitrogen and molecular oxygen.

12. Sterilisation process according to any one of claims 1 to 10 wherein the gas stream includes at least two other gases in addition to nitrogen and molecular oxygen.

13. Sterilisation process according to claim 11 or 12 wherein the other gases are chosen from among argon, helium, nitrogen dioxide, xenon, neon and krypton.

14. Process according to any one of claims 1 to 13 wherein the sterilising species includes photons, radicals, atoms and/or molecules.

15. Process according to claim 14 wherein the photon and/or radical population is the main one.

16. Process according to any one of claims 1 to 15 wherein the objects are contaminated by microorganisms such as viruses, spores, bacteria, fungi, moulds and prions.

17. Process according to any one of claims 1 to 16 wherein the temperature in the sterilisation chamber is 60° Celsius or less, preferably this temperature is around 30° Celsius.

18. Process according to any one of claims 1 to 17 of a duration between 10 minutes and 4 hours.

19. Process according to any one of claims 1 to 18 carried out individually or repetitively in a sequential process consisting of several steps.

20. Process according to claim 19 wherein the steps involve a pulsed gas in an electrical filed applied continuously, a pulsed electrical field in a continuous gas stream, a pulsed gas in a pulsed electrical field in a synchronous manner, a gas change or a combination of these steps.

## Patentansprüche

1. Verfahren zum Sterilisieren von verunreinigten Gegenständen, bei dem die Gegenstände in einer Sterilisationskammer einem Plasma ausgesetzt werden, das in wenigstens einer in die Kammer führenden Entladungsleitung aus einem molekularen Sauerstoff enthaltenden Stickstoffgasstrom erzeugt wird, wobei das Plasma sterilisierende Spezies enthält, die erzeugt werden, während der Gasstrom einem elektrischen Feld ausgesetzt wird, das stark genug ist, um das Plasma zu erzeugen,
wobei das Verfahren umfaßt, die verunreinigten Gegenstände den sterilisierenden Spezies auszusetzen, wobei das Aussetzen in einem Nachentladungsbereich oder in einem Plasmaerregungsbereich stattfindet, Verfahren, bei dem:
- der Prozentsatz des molekularen Sauerstoffs in dem Stickstoffgasstrom auf einen Gehalt x an molekularem Sauerstoff, wie 0 < x < 0,5 % eingestellt wird, wobei x vorzugsweise zwischen 0,1 und 0,4 % variiert, und eine UV-Strahlung maximaler Stärke **dadurch** erhalten wird, daß die Durchflußmenge des Gasstroms für einen gegebenen Druck eingestellt wird oder daß der Druck des Gasstroms für eine gegebene Durchflußmenge eingestellt wird,
- der molekulare Sauerstoff wenigstens teilweise in atomaren Sauerstoff umgewandelt wird, und
- der Querschnitt der Entladungsleitung an ihrem Eintritt in die Sterilisationskammer (SCD) und der Querschnitt der Sterilisationskammer (SCS) die Beziehung 0,05 < (SCD)/(SCS) < 0,7 erfüllen,
wobei der Querschnitt (SCD) den Querschnitt der Entladungsleitung in Kontakt mit der Sterilisationskammer darstellt, der senkrecht zur Richtung des die Entladungsleitung beaufschlagenden Gasstroms ist, und wobei der Querschnitt (SCS) den Querschnitt der Kammer in Kontakt mit der Entladungsleitung darstellt, welcher senkrecht zum Plasmastrom ist.

2. Verfahren nach Anspruch 1, bei dem 0,09 ≤ R ≤ 0,60, vorzugsweise 0,15 ≤ R ≤ 0,5.

3. Verfahren nach Anspruch 2, bei dem 0,2 ≤ R ≤ 0,40, R vorzugsweise nahe 0,25 ist.

4. Verfahren nach einem der Ansprüche 1 bis 3, bei dem das elektrische Feld, welches das Plasma erzeugt, ein Hochfrequenzfeld ist.

5. Verfahren nach Anspruch 4, bei dem die Frequenz des elektrischen Feldes zwischen 10 Megahertz und 3 Gigahertz liegt, vorzugsweise die Frequenz zwischen 100 und 2450 MHz schwankt.

6. Verfahren nach Anspruch 5, bei dem die Frequenz 200 bis 915 MHz beträgt.

7. Verfahren nach einem der Ansprüche 1 bis 6, bei dem die Durchflußmenge des Versorgungsgasstroms einen Wert aufweist, der zwischen 10 und 5000 Standardkubikzentimeter pro Minute liegt.

8. Verfahren nach Anspruch 7, bei dem die Durchflußmenge des Versorgungsgasstroms 50 bis 3000 Standardkubikzentimeter pro Minute beträgt.

9. Verfahren nach einem der Ansprüche 1 bis 8, bei dem der innerhalb der Sterilisationskammer erzeugte Druck zwischen 13,3 und 1330 Pa (0,1 und 10 Torr) liegt.

10. Verfahren nach Anspruch 9, bei dem der innerhalb der Sterilisationskammer erzeugte Druck zwischen 133 und 1064 Pa (1 und 8 Torr) liegt.

11. Sterilisationsverfahren nach einem der Ansprüche 1 bis 10, bei dem der Versorgungsgasstrom zusätzlich zu dem Stickstoff und dem molekularen Sauerstoff wenigstens ein weiteres Gas enthält.

12. Sterilisationsverfahren nach einem der Ansprüche 1 bis 10, bei dem der Gasstrom zusätzlich zu dem Stickstoff und dem molekularen Sauerstoff wenigstens zwei weitere Gase enthält.

13. Sterilisationsverfahren nach Anspruch 11 oder 12, bei dem die weiteren Gase unter Argon, Helium, Stickstoffdioxid, Xenon, Neon und Krypton ausgewählt sind.

14. Verfahren nach einem der Ansprüche 1 bis 13, bei dem die sterilisierenden Spezies Photonen, Radikale, Atome und/oder Moleküle umfassen.

15. Verfahren nach Anspruch 14, bei dem die Photonen- und/oder Radikalenpopulation in der Mehrheit vorliegt.

16. Verfahren nach einem der Ansprüche 1 bis 15, bei dem die Gegenstände durch Mikroorganismen, wie Viren, Sporen, Bakterien, Pilze, Schimmelpilze oder durch Prionen verunreinigt sind.

17. Verfahren nach einem der Ansprüche 1 bis 16, bei dem die Temperatur in der Sterilisationskammer 60° Celsius oder weniger beträgt und vorzugsweise diese Temperatur bei etwa 30° Celsius liegt.

18. Verfahren nach einem der Ansprüche 1 bis 17, mit einer Dauer zwischen 10 Minuten und 4 Stunden.

19. Verfahren nach einem der Ansprüche 1 bis 18, das isoliert oder wiederholt in einem sequentiellen Prozeß mit mehreren Schritten durchgeführt wird.

20. Verfahren nach Anspruch 19, bei dem die Schritte ein gepulstes Gas in einem kontinuierlich angelegten elektrischen Feld, ein gepulstes elektrisches Feld in einem kontinuierlich strömenden Gas, ein gepulstes Gas in einem synchron gepulsten elektrischen Feld, einen Gaswechsel oder eine Kombination dieser Schritte verwenden.
